(19) Europäisches Patentamt
European Patent Office
Office européen des brevets

(11) Veröffentlichungsnummer: 0 169 470
A2

# (12) EUROPÄISCHE PATENTANMELDUNG

(21) Anmeldenummer: 85108806.2

(22) Anmeldetag: 15.07.85

(51) Int. Cl.⁴: A 61 K 45/06
A 61 K 31/44, A 61 K 31/535
//(A61K31/44, 31:18, 31:165, 31:135), (A61K31/535, 31:44)

(30) Priorität: 25.07.84 DE 3427402

(43) Veröffentlichungstag der Anmeldung: 29.01.86 Patentblatt 86/5

(84) Benannte Vertragsstaaten: AT BE CH DE FR GB IT LI NL SE

(71) Anmelder: BAYER AG
Konzernverwaltung RP Patentabteilung
D-5090 Leverkusen 1 Bayerwerk(DE)

(72) Erfinder: Gross, Rainer, Dr.
Platzhoffstrasse 23
D-5600 Wuppertal 1(DE)

(72) Erfinder: Kazda, Stanislav, Dr.
Gellertweg 18
D-5600 Wuppertal 1(DE)

(72) Erfinder: Schlüter, Gerhard, Prof. Dr. med.
Gellertweg 35
D-5600 Wuppertal 1(DE)

(72) Erfinder: Schramm, Matthias, Dr.
Paffrather Strasse 38
D-5000 Köln 80(DE)

## (54) Neues Kombinationspräparat, Verfahren zu seiner Hestellung und seine Verwendung als Arzneimittel.

(57) Die Erfindung betrifft eine neue Wirkstoffkombination mit antihypertensiver und antianginöser Wirkung, enthaltend ein Dihydropyridin der allgemeinen Formel 1

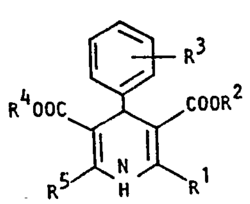

(I)

in welcher $R^1$ bis $R^5$ die in der Beschreibung angegebene Bedeutung haben und einen β-Blocker ohne lokalanästhetische Wirkung.

EP 0 169 470 A2

Croydon Printing Company Ltd.

BAYER AKTIENGESELLSCHAFT 5090 Leverkusen, Bayerwerk

Konzernverwaltung RP
Patentabteilung KS/bo/c

III (PH)

# Neues Kombinationspräparat, Verfahren zu seiner Herstellung und seine Verwendung als Arzneimittel

Die Erfindung betrifft eine neue Wirkstoffkombination mit antihypertensiver und antianginöser Wirkung, enthaltend teilweise bekannte Dihydropyridine (Komponente A) und bestimmte ß-Blocker (Komponente B), die eine lange Wirkungsdauer und gute Verträglichkeit besitzt.

Es ist bereits bekannt, Kombinationen von verschiedenen ß-Blockern mit verschiedenen Calciumantagonisten bei der Behandlung von Kreislauferkrankungen, insbesondere von Hypertonie und Koronarerkrankungen einzusetzen. Aus den Wirkungsmechanismen von Kalzium-Antagonisten und von ß-Blockern kann man jedoch ein im Vergleich zur Monotherapie höheres Risiko im Hinblick auf mögliche unerwünschte Nebenwirkungen ableiten. Zum Beispiel wird von V.S. Wayne, et al., in Australien N.Z.J.Med. 12, Nr. 4, 285 - 289 (1982) über schwere Nebenwirkungen der Kombination zwischen dem Calciumantagonisten Verapamil und den ß-Blockern Metoprolol und Pindolol berichtet. Auch

M.D. Winniford et al., (Zirkulation 66, Nr. 4 Pt. 2, Page 120 (1982) Dallas USA) berichten über mögliche schwere Nebenwirkungen im Zusammenhang mit der Behandlung von 13 Anginapatienten mit einer Kombination des ß-Blokkers Propanolol und des Calciumantagonisten Verapamil. Demgegenüber zeichnen sich die erfindungsgemäß ausgewählten Kalzium-Antagonisten aus der Gruppe der 1,4-Dihydropyridine durch eine in dieser Hinsicht deutlich bessere Verträglichkeit aus; bei Wahl geeigneter ß-Blocker als Kombinationspartner wird das Nutzen-Risiko-Verhältnis weiter optimiert.

Die vorliegende Erfindung betrifft ein neues antihypertensives und antianginöses Kombinationspräparat enthaltend eine Kombination aus einem Dihydropyridin mit unsymmetrischer Estergruppierung (Komponente A) der allgemeinen Formel I

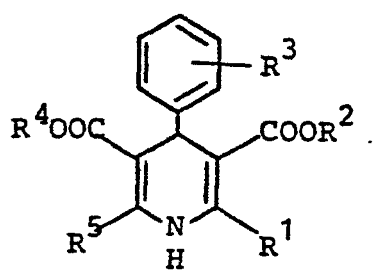

(I)

in welcher

$R^1$ und $R^5$ gleich oder verschieden sind und für Methyl, Hydroxymethyl oder Cyano stehen,

$R^2$ und $R^4$ immer voneinander verschieden sind und für Alkyl mit 1 bis 10 Kohlenstoffatomen stehen, welches gegebenenfalls substituiert ist durch Alkoxy mit 1 bis 4 Kohlenstoffatomen, Fluor, Chlor oder N-Methyl-benzylamino, und

$R^3$ für 1 oder 2 gleiche oder verschiedene Substituenten steht aus der Gruppe Nitro, Chlor und Trifluormethyl oder für den Rest =N-O-N= steht,

und einem ß-Blocker ohne lokalanästhetische Wirkung in einem Gewichtsverhältnis von 0,1 bis 10 Gewichtsteile der Komponente A und 10 bis 200 Gewichtsteile der Komponente B.

Die Erfindung betrifft vorzugsweise ein antihypertensives und antianginöses Kombinationspräparat, welches als Komponente A das Dihydropyridin 1,4-Dihydro-2,6-dimethyl-4-(2-nitrophenyl)-pyridin-3,5-dicarbonsäure-isobutyl-methylester (Nisoldipin) oder das Dihydropyridin 2,6-Dimethyl-4-(3'-nitrophenyl)-1,4-dihydropyridin-3,5-dicarbonsäure-3-methylester-5-ethylester (Nitrendipin), enthält und als Komponente B einen ß-Blocker aus der Gruppe Atenolol, Sotalol, Timolol oder Nadolol enthält.

Die beiden Dihydropyridine sowie ihre Blutdruck- und Koronarwirkung sind bekannt (vgl. DOS 2 117 571; Brit.-Pat. 1 358 951; DOS 2 549 568; Brit.-Pat. 1 516 793). Ihre Verwendung als Kombinationspartner mit den genannten ß-Blockern ist neu.

Die erfindungsgemäß verwendbaren ß-Blocker ohne lokalanästhetische Wirkung sind ebenfalls bekannt (vgl. Uloth et al., J. Med. Chem. 9, 88 (1966), US-Patente 3 663 607, 3 836 671, 3 619 370, 3 655 663, 3 657 237, 3 935 267).

Ihre Verwendung als Kombinationspartner mit den genannten unsymmetrischen Dihydropyridinen ist neu.

Die erfindungsgemäß genannten ß-Blocker sind ausgewählt aus der großen Gruppe bekannter ß-Blocker. Im Stand der Technik gibt es zahlreiche Hinweise dafür, daß Kombinationen von ß-Blockern mit vielen Calciumantagonisten mit dem Risiko unerwünschter Nebenwirkungen behaftet sind und somit für die Therapie von Koronar- und Kreislauferkrankungen nur begrenzt eingesetzt werden können. Die Kenntnis der Mechanismen der Automatie in Herzschrittmacher-Gewebe und der Einflüsse von Calciumantagonisten und ß-Blockern hierauf zeigt, daß es z.B. bei mit ß-Blockern behandelten Tieren zum Ausfall der Spontan-Erregung des Herzschrittmacher-Gewebes kommen kann, wenn gleichzeitig höhere Dosen von Calciumantagonisten verabreicht werden.

Es war nicht vorhersehbar, daß eine Kombination von ausgewählten ß-Blockern ohne lokalanästhetische oder auch Natriumantagonistische Wirkung mit den erfindungsgemäß ausgewählten Kalzium-Antagonisten aus der Gruppe der 1,4-Dihydropyridine eine so vorteilhafte Wirkung zeigt und sich insbesondere durch gute Verträglichkeit auszeichnet.

Es ist daher ausgesprochen überraschend, daß die erfindungsgemäßen Kombinationen von ausgewählten Kalzium-Antagonisten mit ausgewählten ß-Blockern ohne lokalanästhetische (Natrium-antagonistische) Wirkung bei vorteilhafter antihypertensiver und antianginöser Wirkung wesentlich besser verträglich sind als Kombinationen mit anderen ß-Blockern, die eine lokalanästhetische bzw. Natriumantagonistische Wirkung aufweisen.

Dies gilt umso mehr als alle bekannten elektrophysiologischen Untersuchungen dafür sprechen, daß das Natriumsystem bei der Automatie der Schrittmacherzellen des Herzens keine Rolle spielt (vergl. z. B. Brown, H.F., Physiol. Rev. 62:505-530 (1982)).

Bei der erfindungsgemäßen Kombination werden zweckmäßigerweise 1 Gew.-Teil der Komponente A mit 1 bis 10, insbesondere 2 bis 6 Gewichtsteilen der Komponente B zusammengegeben. Die Tagesdosierung der erfindungsgemäßen Kombination enthält zweckmäßigerweise 1 bis 100 mg, vorzugsweise 2 bis 50 mg, insbesondere 5 bis 30 mg der Komponente A und 1 bis 200 mg, vorzugsweise 10 bis 100 mg, insbesondere 30 bis 80 mg der Komponente B.

Das erfindungsgemäße Kombinationspräparat wird vorzugsweise als feste Zubereitung, z.B. in Form von Tabletten, Pillen, Dragees, Granulaten, Pulvern, Kapseln oder Sachets zur Anwendung gebracht.

Die Herstellung der galenischen Formulierungen kann in bekannter Weise erfolgen, unter Verwendung bekannter Hilfs- und Trägerstoffe und gegebenenfalls unter Hinzugabe weiterer geeigneter Wirkstoffe wie Saluretika, insbesondere der Wirkstoffe Mefrusid oder Muzolimin.

Besonders hervorgehoben seien solche Kombinationspräparate, in denen der Wirkstoff der Komponente A in einer Retardformulierung eingesetzt wird, wie z.B. in Form von Wirkstoffkristallen mit der spezifischen Oberfläche 0,5 bis 4 $m^2$/g. Eine weitere Möglichkeit für die Komponente A ist der Einsatz eines Dihydropyridins, das zu 60 bis 100 % in kristalliner Form vorliegt und zu 0 bis 40 % in amorpher Form vorliegt, z.B. als Copräzipitat mit Polyvinylpyrrolidon (PVP), Methylcellulose, Hydroxypropylcellulose oder Hydroxypropylmethylcellulose .

Durch die Verwendung eines Gemisches von amorphem Dihydropyridin und kristallinem Dihydropyridin wird der Vorteil eines schnellen Wirkungseintritts mit dem Vorteil einer langen Wirkungsdauer vereinigt.

Die erfindungsgemäßen Kombinationspräparate besitzen eine überraschend gute Verträglichkeit. Während bei Ratten, die mit Propranolol ß-blockiert wurden, EKG-Veränderungen (Ausfall der P-Welle) nach intravenöser Infusion einer kummulativen Dosis von Nitrendipin von 2,5 mg/kg (Infusionsrate 300 µg/kg/min) auftreten, wird bei entsprechend ß-blockierten Ratten mit dem erfindungsgemäßen ß-Blocker Sotalol eine Dosis von 12,2 mg/kg Nitrendipin toleriert. Das heißt, die erfindungsgemäße Kombination Nitrendipin und Sotalol ist ca. 5-fach verträglicher als die Kombination Nitrendipin/Propranolol. Die erfindungsgemäße Kombination Atenolol/Nitrendipin zeigt in der gleichen Versuchsanordnung eine ca. 3-fach verbesserte Verträglichkeit.

Neben der unerwartet guten Verträglichkeit der erfindungsgemäßen Kombination, die zu einer deutlichen Minderung des Risikos des zu behandelnden Patienten führt, erleichtert die Verabreichung der erfindungsgemäßen fixen Kombination die Handhabung durch den Patienten (verbesserte Patient compliance).

Weitere Vorteile der erfindungsgemäßen Kombination sind darin zu sehen, daß im Falle schwerer Kreislauferkrankungen, insbesondere starkem Hochdruck oder koronarer Herzkrankheit, wenn eine Therapie mit Monopräparaten nicht mehr erfolgreich ist, das erfindungsgemäße Kombinationsprodukt bequem und sicher angewandt werden kann. Auch bei eingeschränktem Handlungsvermögen des Patienten ist die Einnahme beider Wirkstoffe in kombinierter Form in richtiger Dosierung sichergestellt und für den behandelnden Arzt leichter kontrollierbar.

Die folgenden Formulierungsbeispiele sollen den Gegenstand der Erfindung verdeutlichen.

Beispiel 1

20 mg Nitrendipin, bestehend aus 15 mg mikrokristallinem Wirkstoff mit einer spezifischen Kristalloberfläche von ca. 3 $m^2$/g und 5 mg Nitrendipin-Copräzipitat mit PVP, werden zusammen mit 50 mg kristallinem Atenolol, 370 mg mikrokristalliner Cellulose, 85 mg Lactose und 7 mg Magnesiumstearat vermischt und zu Tabletten gepreßt.

Beispiel 2

20 mg Nitrendipin, bestehend aus 5 mg mikrokristallinem Wirkstoff mit einer spezifischen Kristalloberfläche von ca. 3 $m^2$/g und 15 mg Nitrendipin-Copräzipitat mit PVP, werden zusammen mit 25 mg kristallinem Atenolol, 150 mg mikrokristalliner Cellulose, 110 mg Maisstärke, 30 mg Crospovidone und 5 mg Magnesiumstearat vermischt und zu Tabletten verpreßt.

Beispiel 3

30 mg Nitrendipin, bestehend aus 10 mg mikrokristallinem Wirkstoff mit einer spezifischen Kristalloberfläche von ca. 3 $m^2$/g und 20 mg Nitrendipin-Copräzipitat mit PVP, werden mit 60 mg kristallinem Timolol, 200 mg mikrokristalliner Cellulose, 150 mg Maisstärke, 35 mg Crospovidone und 5 mg Magnesiumstearat vermischt und zu Tabletten gepreßt.

Beispiel 4 (nur für Nitrendipin)

50 mg Nitrendipin als mikrokristalliner Wirkstoff mit einer spezifischen Kristalloberfläche von ca. 3,5 $m^2/g$ werden mit 100 mg kristallinem Timolol, 50 mg mikrokristalliner Cellulose, 70 mg Maisstärke, 26 mg Lactose, 20 mg PVP, 2 mg Natriumlaurylsulfat und 2 mg Magnesiumstearat vermischt und zu Tabletten gepreßt.

Beispiel 5

10 mg Nitrendipin als mikrokristalliner Wirkstoff mit einer spezifischen Kristalloberfläche von ca. 3 $m^2/g$ werden mit 50 mg kristallinem Sotalol, 27,5 mg Maisstärke, 20 mg mikrokristalliner Cellulose, 16 mg Calciumhydrogenphosphat, 5 mg PVP, 1 mg Polysorbat und 0,5 mg Magnesiumstearat vermischt und zu Tabletten gepreßt.

Beispiel 6

20 mg Nitrendipin als mikrokristalliner Wirkstoff mit einer spezifischen Kristalloberfläche von ca. 3 $m^2/g$ werden mit 30 mg kristallinem Nadolol, 28,5 mg Maisstärke, 25 mg mikrokristalliner Cellulose, 20 mg Magnesiumcarbonat, 5 mg PVP, 1 mg Natriumlaurylsulfat und 0,5 mg Magnesiumstearat vermischt und zu Tabletten verpreßt.

Beispiel 7

20 mg Nisoldipin als mikrokristalliner Wirkstoff mit einer spezifischen Kristalloberfläche von ca. 2,5 $m^2/g$ werden mit 120 mg kristallinem Atenolol, 80 mg mikrokristalliner Cellulose, 66 mg Maisstärke, 40 mg Lactose, 10 mg PVP, 2 mg Natriumlaurylsulfat und 2 mg Magnesiumstearat vermischt und zu Tabletten verpeßt.

Beispiel 8

10 mg Nitrendipin als mikrokristalliner Wirkstoff mit einer spezifischen Kristalloberfläche von ca. 2,5 $m^2/g$ werden mit 60 mg kristallinem Sotalol, 40 mg mikrokristalliner Cellulose, 33 mg Maisstärke, 20 mg Lactose, 5 mg PVP, 1 mg Natriumlaurylsulfat und 1 mg Magnesiumstearat vermischt und zu Tabletten gepreßt.

Beispiel 9

5 mg Nitrendipin als mikrokristalliner Wirkstoff mit einer spezifischen Kristalloberfläche von ca. 3 $m^2/g$ werden mit 40 mg kristallinem Nadolol, 30 mg mikrokristalliner Cellulose, 29 mg Maisstärke, 20 mg Magnesiumcarbonat, 5 mg PVP, 0,5 mg Polysorbat und 0,5 mg Magnesiumstearat vermischt und zu Tabletten gepreßt.

Beispiel 10

1 mg Nitrendipin als mikrokristalliner Wirkstoff mit einer spezifischen Kristalloberfläche von ca. 2,5 $m^2/g$

werden mit 70 mg kristallinem Timolol, 20 mg mikrokristalliner Cellulose, 15 mg Calciumhydrogenphosphat, 8 mg Maisstärke, 0,5 mg Natriumlaurylsulfat und 0,5 mg Magnesiumstearat vermischt und zu Tabletten gepreßt.

Beispiel 11 (nur für Nisoldipin)

0,5 mg Nisoldipin als mikrokristalliner Wirkstoff mit einer spezifischen Kristalloberfläche von ca. 3,5 $m^2/g$ werden mit 25 mg kristallinem Atenolol, 20 mg Cellulosepulver, 10 mg Lactose, 19,7 mg Maisstärke, 4 mg PVP, 0,5 mg Natriumlaurylsulfat und 0,3 mg Magnesiumstearat vermischt und zu Tabletten gepreßt.

Patentansprüche

1. Antihypertensives und antianginöses Kombinationspräparat enthaltend eine Kombination aus einem Dihydropyridin mit unsymetrischer Estergruppierung (Komponente A) der allgemeinen Formel I

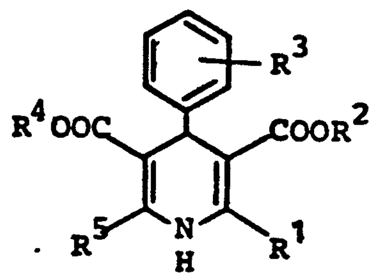

(I)

in welcher

$R^1$ und $R^5$ gleich oder verschieden sind und für Methyl, Hydroxymethyl oder Cyano stehen,

$R^2$ und $R^4$ immer voneinander verschieden sind und für Alkyl mit 1 bis 10 Kohlenstoffatomen stehen, welches gegebenenfalls substituiert ist durch Alkoxy mit 1 bis 4 Kohlenstoffatomen, Fluor, Chlor oder N-Methyl-benzylamino und

$R^3$ für 1 oder 2 gleiche oder verschiedene Substituenten steht aus der Gruppe Nitro, Chlor und Trifluormethyl oder für den Rest =N-O-N=

und einem ß-Blocker ohne lokalanästhetische Wirkung (Komponente B) in einem Gewichtsverhältnis von 0,1 bis 10 Gewichtsteile der Komponente A und 10 bis 200 Gewichtsteile der Komponente B.

2. Antihypertensives und antianginöses Kombinationspräparat gemäß Anspruch 1, dadurch gekennzeichnet, daß als Komponente B ein ß-Blocker aus der Gruppe Atenolol, Sotalol, Timolol oder Nadolol eingesetzt wird.

3. Antihypertensives und antianginöses Kombinationspräparat gemäß Anspruch 1, dadurch gekennzeichnet, daß als Komponente A Nitrendipin oder Nisoldipin eingesetzt werden.

4. Kombinationspräparat gemäß Ansprüchen 1 und 2, dadurch gekennzeichnet, daß die Komponente A und B in einem Gewichtsverhältnis von 1:2-6 eingesetzt werden.

5. Kombinationspräparat gemäß den Ansprüchen 1 bis 4, dadurch gekennzeichnet, daß eine Tagesdosis 1 bis 100 mg der Komponente A und 1 bis 200 mg der Komponente B enthält.

6. Kombinationspräparat gemäß den Ansprüchen 1 bis 4, dadurch gekennzeichnet, daß es 5 bis 30 mg der Komponente A und 30 bis 80 mg der Komponente B enthält.

7. Kombinationspräparat gemäß den Ansprüchen 1 bis 6 in Form von Pulvern, Granulaten, Tabletten, Kapseln oder Dragees.

8. Verfahren zur Herstellung von Kombinationspräparaten gemäß den Ansprüchen 1 bis 7, dadurch gekennzeichnet, daß man die Komponente A und die Komponente B gemeinsam mit Hilfs- und Trägerstoffen, gegebenenfalls unter Zugabe weiterer Wirkstoffe, in eine geeignete Applikationsform überführt.

9. Verfahren zur Herstellung von Kombinationspräparaten gemäß Anspruch 8, dadurch gekennzeichnet, daß man die Komponente A in kristalliner oder amorpher Form einsetzt.